# EUROPEAN PATENT SPECIFICATION

(11) **EP 1 962 778 B1**
(45) Date of publication and mention of the grant of the patent: **23.08.2017**
(21) Application number: 06840343.5
(22) Date of filing: 21.12.2006
(51) Int. Cl.: A61K 8/27, A61K 8/365, A61Q 11/00, A61K 31/194, A61K 33/30, A61K 8/24, A61K 8/81, A61K 9/00, A61K 8/19, A61K 31/315, A61K 33/00, A61K 8/67

(54) **IMPROVED ORAL COMPOSITIONS COMPRISING ZINC CITRATE**
VERBESSERTE ORALE ZUSAMMENSETZUNGEN MIT ZINKCITRAT
COMPOSITIONS ORALES AMELIOREES COMPRENANT DU CITRATE DE ZINC

(30) Priority: 21.12.2005 US 752341 P
(43) Date of publication of application: 03.09.2008
(73) Proprietor: Colgate-Palmolive Company, New York, NY 10022 (US)
(72) Inventor: PRENCIPE, Michael, Princeton Junction, NJ 08550 (US); FRUGE, Linh, Hillsborough, NJ 08844 (US); MELLO, Sarita V., Somerset, NJ 08873 (US); GAFFAR, Abdul, Princeton, NJ 08540 (US)
(74) Representative: Wibbelmann, Jobst
(86) International application number: PCT/US2006/062499
(87) International publication number: WO 2007/076444

(56) References cited:
- EP-A- 0 095 871
- EP-A- 0 657 160
- EP-A- 1 595 537
- WO-A-02/45678
- WO-A-94/14407
- WO-A-94/26258
- WO-A-03/015494
- WO-A-03/017963
- WO-A-2004/045446
- WO-A-2005/092277
- US-A- 4 325 939
- US-A- 5 948 390
- US-A1- 2004 010 429
- US-A1- 2005 096 383
- US-B1- 6 221 340
- DATABASE WPI Week 200534 Derwent Publications Ltd., London, GB; AN 2005-326486 XP002436441 & JP 2005 112852 A (SUNSTAR CHEM IND CO LTD) 28 April 2005 (2005-04-28)

## Description

### BACKGROUND OF THE INVENTION

Dental plaque is generally believed to be formed as a byproduct of bacterial growth, and comprises a dense microbial layer containing a mass of microorganisms embedded in a polysaccharide matrix that adheres to surfaces of teeth and at the gingival margin. Periodontal diseases are inflammatory disorders that are the result of complex interactions between periodontopathogens and the host's immune system response. Gingivitis is the inflammation or infection of the gums and the alveolar bones that support the teeth, and the cause is generally believed to be both bacteria in the mouth (particularly the bacteria associated with plaque formation) and the inflammatory response triggered by the presence of bacteria/ bacterial byproduct toxins. Periodontitis is a progressively worsened state of disease as compared to gingivitis, where inflamed gums begin to recede from the teeth, which can ultimately result in destruction of the bone and periodontal ligament. Chronic infection and inflammation potentially results in the subsequent loss of teeth.

Further, bacteria generate acids as end-products of the bacterial degradation of fermentable carbohydrates. These acids can dissolve hydroxyapatite, which can result in the loss of cementum and/or enamel potentially leading to formation of dental caries or dentinal hypersensitivity. Dentinal hypersensitivity can occur when protective enamel or cementum covering dentin is lost, thereby exposing the dentin tubules to the mouth environment. When the fluid that fills the narrow dentinal tubules is exposed to the mouth, it enables cold, tactile, evaporative and osmotic stimuli to be transmitted through the dentin to the pulp, which is then sensed as sharp pain in the nerve fibers. Dental caries entail the loss of enamel/cementum, typically followed by enzymatic lysis of the underlying tissue, then cavity formation that can penetrate the enamel, dentin and may reach the pulp, ultimately leading to tissue necrosis.

There is a need for oral care compositions that effectively reduce the development or progression of oral disease, preferably containing an active ingredient that functions to diminish the effects of oral disease by preventing or reducing multiple etiological factors that contribute to and/or exacerbate oral disease. There is a continuing interest in the oral care field for oral care compositions that improve the treatment of both plaque and tartar formation. Furthermore, it is desirable to have oral care compositions that provide multiple treatment benefits, such as those oral compositions that also reduce dentinal sensitivity, caries formation, and/or inflammation in the oral cavity.

WO-A-02/45678 discloses a zinc containing dentifrice of reduced astringency

### BRIEF SUMMARY OF THE INVENTION

In certain embodiments, the invention is directed to an antiplaque and desensitizing oral composition comprising: an active ingredient comprising a zinc citrate agent and a potassium citrate agent; and a copolymer of maleic anhydride and vinyl methyl ether, and a polyphosphate.

In certain embodiments, the present invention is directed to a an oral composition of the present invention for use in a method of treating an oral surface having dental sensitivity, the method comprising contacting the oral surface with the oral composition.

### DETAILED DESCRIPTION OF THE INVENTION

As used throughout the present disclosure, ranges are a shorthand for describing each and every value that is within the range. Any value within the range can be elected as the terminus of the range. Where there is a conflict between a definition in the present disclosure and that of a cited reference, the present disclosure controls.

In various embodiments, the present invention provides methods of treating an oral cavity having an oral surface, and oral compositions comprising an active ingredient comprising a zinc ion source.

In certain embodiments, the oral compositions reduce formation of at least one of plaque and tartar, or both, on the oral surface. In certain embodiments, the oral composition reduces or mitigates gingivitis or periodontitis for patients presenting symptoms of such a disease. In certain embodiments, the oral compositions reduce sensitivity in an oral surface, for example, a tooth having dentinal sensitivity. In other embodiments, the methods and oral compositions reduce inflammation of oral surfaces, *e.g.,* oral tissues.

As used herein, an "oral surface" includes the hard and soft tissues of the oral cavity. As used herein, "hard tissues" refers to tissues such as the teeth and periodontal support in an oral cavity, such as that of a mammal. "Soft tissues" refers to tissues such as the gums, the tongue, the surfaces of the buccal cavity and the like. For example, dentinal sensitivity is typically associated with hard tissue, *i.e.*, a tooth, in particular, dentin tissue in the root region covered by cementum and/or dentin in the crown region of a tooth is covered by enamel. In various embodiments, the methods and compositions of the present invention treat various oral surfaces in the oral cavity, and can include treatment of both hard and soft tissues simultaneously, for example, reducing inflammation in soft tissues and preventing plaque, tartar, caries and/or sensitivity in the hard tissues.

In various embodiments, the present oral compositions treat and/or inhibit various oral inflammatory conditions, such as gingivitis, periodontitis, oral lichen planus, Sjögren's syndrome, and the like. The oral compositions can be present in various different forms, including a dentifrice, paste, gel, medicament, powder, mouthrinse, mouthwash, tooth hardener, oral film, slurry, injectable solution, and lozenge, as well as any other form of oral care compositions known in the art.

In various embodiments, the oral composition comprises an active ingredient that comprises a zinc ion source. Exemplary suitable zinc ion sources for use in the present embodiments include zinc salts such as, *e.g.,* zinc citrate, sodium zinc citrate, zinc acetate, zinc gluconate, zinc glycinate, zinc oxide, zinc sulfate, zinc bacitracin, zinc tribromosalicylanilide, zinc carbonate, zinc fluoride, zinc formate, zinc lactate, zinc oleate, zinc peroxide, zinc phosphate, zinc pyrophosphate, zinc silicate, zinc stearate, zinc tannate, zinc oxalate, zinc chloride or mixtures thereof.

In the present ivention, the zinc ion source is a zinc citrate agent. As used herein, the term "agent" refers to a composition that delivers an active compound having the desired biological, physiological, chemical and/or mechanical effects. An agent may include precursors of the active compound that form the active compound in *vivo* or the agent may comprise the active compound and/or homologues, analogues, or derivatives thereof. The agents can contain active compounds that are natural or synthetic. As appreciated by one of skill in the art, the agent may further comprise other components that do not detract from the efficacy of the active compound, for example, buffers, diluents, and impurities may be present in the agent.

In the context of the various embodiments, zinc citrate is useful as an active ingredient because it is particularly efficacious as an antibacterial, antiplaque, antitartar, and antimalodor active agent. It is believed that in addition to providing zinc ions *in vivo,* the citrate anion in particular contributes to the anti-bacterial efficacy of active ingredient, and as such is particularly efficacious in oral care compositions.

Thus, the zinc citrate agent can include a zinc citrate active compound having a general formula of Zn₃(C₆H₅O₇)₂. Zinc citrate can be provided in an analogous hydrated form, for example, it is commonly available as zinc citrate trihydrate Zn₃(C₆H₅O₇)₂·3H₂O or zinc citrate dihydrate Zn₃(C6H₅O₇)₂·2H₂O. Also, zinc citrate is sometimes provided as sodium zinc citrate Na₂Zn₂(C₆H₅O₇)₂. Zinc citrate active compound can be provided in an oral composition in several ways; for example, it can be formed from the precursors citric acid and zinc chloride, zinc carbonate, or other common zinc salts that combine with the citric acid to form the desired zinc citrate compound.

In certain embodiments, the invention provides oral compositions that treat an oral surface having dentinal sensitivity. Dentinal sensitivity (also known as dentin hypersensitivity) typically occurs where protective enamel or cementum covering dentin of a tooth is lost. For example, a breach of the cementum typically occurs when there is gingival tissue recession that permits exposure of the cementum to the oral cavity, thus making the cementum susceptible to dissolution. Dentinal sensitivity often causes pain when the exposed area of the tooth (*i.e.,* dentin) comes into contact with cold or hot temperatures, high concentrations of acid or sugars, or metals. Various methods of reducing dental sensitivity have been employed. It is generally recognized that an effective treatment for treating dentinal sensitivity is the effective and regular removal of plaque. The frequent and regular removal of plaque allows remineralization of the exposed dentinal tubules from salivary minerals, thus alleviating much of the underlying cause of pain. However, this method of treatment is not always an effective course of treatment in and of itself, as it is often not rapid enough to alleviate pain, or the plaque removal regimen is too rigorous or ineffective for a subject experiencing dentinal sensitivity.

Often, treatment for dentinal sensitivity entails application of an active ingredient comprising a desensitizing agent via an oral composition, such as a dentifrice or medicament. The use of desensitizing agents in oral compositions is known, including by way of example, active ingredients such as stannous and sodium fluoride, potassium, lithium or sodium nitrate, sodium fluoride, sodium monofluorophosphate, strontium chloride, potassium tartrate, potassium chloride, potassium sulfate, sodium and potassium citrate, and mixtures thereof.

In certain embodiments, the oral composition comprises an active ingredient that comprises a desensitizing active agent in addition to the zinc citrate agent. In such oral compositions, the treatment may reduce sensitivity of the oral surface, preferably that of the teeth. By reducing sensitivity, it is meant that pain and/or discomfort associated with the dentinal sensitivity is noticeably reduced and preferably is eliminated such that the user has little or no perception of pain and/or discomfort. In certain embodiments, the desensitizing agent comprises an alkali metal or alkaline earth metal cation complexed with a citrate anion. The alkali earth metal or alkaline earth metal citrate agents may include, for example, potassium or sodium citrate compounds.

In the present invention, a potassium citrate agent is used as a desensitizing agent in the oral compositions. The potassium citrate compound has a general formula of K₃C₆H₅O₇, which can also be hydrated. While not limiting to the present invention, it is believed that alkali metal cations of citrate anions, in particular potassium cations, chemically interfere with the transmission of pain signals generated by the pulpal nerve fibers of the exposed tubules. Further, as described above, the citrate anion is believed to contribute to the anti-bacterial efficacy of the overall active ingredient, thus reducing the formation of plaque and tartar, *inter alia,* which when unabated can further contribute to the underlying etiology of dentin hypersensitivity, namely erosion of the mineralized layer over dentin.

In various embodiments, the oral compositions may further comprise one or more additional desensitizing agents. In certain embodiments, such additional desensitizing agents (in addition to a potassium citrate agent) may include compounds known in the art, including those described above. In certain embodiments, the active ingredient comprises a potassium citrate agent and a second desensitizing agent chosen from potassium tartrate, potassium chloride, potassium sulfate, potassium nitrate, sodium nitrate, sodium citrate or mixtures thereof.

In certain embodiments, the oral compositions comprise a zinc citrate agent and a potassium citrate agent, where the ratio of the zinc citrate agent to the potassium citrate agent is about 1:1 to about 1:5. In some embodiments the ratio of the zinc citrate to the potassium citrate agent is about 1:2 to about 1:3.

In various embodiments, the oral composition comprises an active ingredient where the zinc citrate agent is present in an amount of about 0.001% to about 5% by weight of the oral composition, about 0.01 to about 3%, about 0.1 to about 3%, or about 1 to about 2% by weight of the oral composition.

In various embodiments, the potassium citrate agent is present in an amount of about 0.001 % to about 10% by weight of the oral composition, about 0.1% to about 7%, about 4 % to about 6 %, or about 5.5% by weight of the oral composition.

In various embodiments, a method of treating an oral surface having dentinal sensitivity comprises contacting such a surface with an oral composition comprising an active ingredient comprising a zinc citrate agent and a potassium citrate agent. The particularly efficacious combination of the zinc citrate agent and the potassium citrate agent provides improved methods of treatment for sensitive teeth. For example, the contacting of the oral composition with the oral surface may reduce not only the sensitivity of the oral surface, but also formation of plaque and/or tartar on the oral surfaces, which are believed to contribute to the conditions that cause and/or exacerbate dentinal sensitivity.

Likewise, the combination of the zinc citrate agent and the potassium citrate agent in certain embodiments of the invention may result in an oral composition having less astringency. Oral compositions comprising a zinc ion source as an active ingredient are known to be highly astringent. To provide aesthetically desirable oral compositions, reduction and/or masking of such astringent properties is preferred. Further, in accordance with certain embodiments, as the amount of citrate anion concentration remains the same (contributed by both the potassium citrate and the zinc citrate), the anti-bacterial efficacy remains of a similar level when compared to a comparative composition comprising solely the zinc citrate. As such, the relative concentration of zinc cations can be lower, thus reducing the astringency of the composition while still maintaining desired antibacterial and desensitizing efficacy.

Further, the oral compositions of the present invention further comprise certain components that reduce the astringency of the composition. Such components include a polyphosphate compound and a synthetic anionic linear carboxylate polymer. Methods of reducing astringency with these components are discussed in U.S. Patent No. 5,000,944 to Prencipe et al. and WO02/45678 to Hoic et al.

The astringency of fully or partially soluble zinc salts, such as zinc citrate and the like, is believed to be reduced by the formation of a complex of the polyphosphate compound with zinc ions. Further, the complex can further include a polycarboxylate polymer, as will be described in more detail below. In oral compositions, the polyphosphates are disclosed to provide benefits including tartar inhibition, as well as the reduction of aesthetic negatives like astringency associated with zinc. A linear molecularly dehydrated polyphosphate compound useful in the present invention generally comprises two or more condensed phosphate molecules. Cyclic polyphosphates are generally referred to as metaphosphates. The number of phosphorus atoms in the condensed phosphate molecules can range from two (generally referred to as a pyrophosphate) to infinity (*i.e.,* polyphosphates). Polyphosphate salts are generally employed in the form of their wholly or partially neutralized water soluble alkali metal (*e.g*., potassium, sodium or ammonium salts, and any mixtures thereof). Examples of suitable polyphosphate compounds include as sodium or potassium tripolyphosphates, sodium and potassium hexametaphosphates, disodium dihydrogen pyrophosphate, tetrasodium pyrophosphate, and tetrapotassium pyrophosphate in their unhydrated as well as hydrated forms.

It is believed that a zinc/polyphosphate complex, *e.g*., zinc/pyrophosphate; zinc/ tripolyphosphate; and zinc/hexametaphosphate complexes provide a combined antitartar activity that is more effective than either of the individual active ingredient agents alone (*i.e.,* Zn⁺² and P₂O₇⁻⁴ (pyrophosphate) ions). A range of polyphosphate ion to zinc ion in a molar ratio is, in various embodiments, about 1:1 to about 5:1, or about 2:1 to about 5:1. In various embodiments, the polyphosphate compound is optionally present in an amount of about 0.01 to about 5%, about 1 to about 4%, a about 1.5 to about 3%, or about 2 to 2.5% by weight of the oral composition. In certain embodiments, the polyphosphate compound comprises tetrapotassium pyrophosphate (TKPP). In some embodiments, the TKPP is present in an amount of about 2 to about 3% by weight of the oral composition, for example about 2.5%.

Synthetic anionic linear polycarboxylates can complex with the zinc and polyphosphate compound complex, and are also known as efficacy enhancing agents for certain oral care active ingredients, including antibacterial, anti-tartar or other active agents within the oral composition. Such anionic polycarboxylates are generally employed in the form of their free acids, or partially neutralized or fully neutralized water soluble alkali metal (*e.g*., potassium and preferably sodium) or ammonium salts. The terms "synthetic" and "linear" exclude known thickening or gelling agents comprising carboxymethylcellulose and other derivatives of cellulose and natural gums, and carbopols having reduced solubility due to cross-linkages.

Preferred copolymers are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 5,000,000. One useful copolymer is methylvinylether/maleic anhydride. Examples of these copolymers are available from ISP Corporation under the trade name GANTREZ®, e.g., AN 139 (M.W.1,100,000), AN 119 (M.W. 200,000); S-97 Pharmaceutical Grade (M.W.1,500,000), AN 169 (M.W. 2,000,000), and AN 179 (M.W. 2,400,000); wherein the preferred copolymer is S-97 Pharmaceutical Grade (M.W. 1,500,000). In various embodiments, a synthetic anionic polycarboxylate is included in the oral composition in an amount of about 0.001 to about 5 weight %, about 0.1 to about 2.0 weight %, or about 1.5 by weight %.

In certain embodiments, a method is provided for treating inflammation in oral tissue. In particular, the oral composition preferably reduces inflammation of the oral tissue by reducing one or more mediators of inflammation. Inflammation of the oral tissue generally refers to a localized protective response elicited by injury or destruction of tissues, which serves to destroy, dilute, or sequester both the injurious agent and the injured tissue. In the acute form, it is characterized by pain, heat, redness, swelling, and loss of function, as where chronic inflammation is a slow process that is primarily characterized by the formation of new connective tissue. Chronic inflammation is often a continuation of acute inflammation or a prolonged low-grade form of inflammation (such as that associated with periodontitis or gingivitis) and usually causes permanent tissue damage. Histologically, inflammation involves a complex series of events, including dilation of arterioles, capillaries, and venules, with increased permeability and blood flow; exudation of fluids, including plasma proteins, and leukocytic migration into the inflammatory locus. Inflammation corresponds to enhanced levels of pro-inflammatory cellular mediators (substances that are released from cells), for example, as the result of the interaction of an antigen with an antibody or by the action of antigen with a sensitized lymphocyte.

Cytokines are non-antibody proteins that are released by one cell population on contact with a specific antigen and act as intercellular mediators to elicit a response of a mammal's immune system. "Interleukin" is a term for a group of multifunctional cytokines that are produced by a variety of lymphoid and nonlymphoid cells. Examples of interleukin compounds generated by gingival fibroblasts include interleukin-1β, interleukin-6, and interleukin-8. Certain interleukin compounds appear to stimulate production of arachidonic acid metabolites, such as prostaglandins, leukotrienes, and thromboxanes, which are produced through the cyclooxygenase or lipoxygenase enzyme pathways. These metabolites have been implicated as the prime mediators in gingivitis, periodontitis, osteomyelitis and other inflammatory diseases.

Leukotrienes in particular appear to activate osteoclasts and inhibitors of osteoblast activity and promote bone resorption associated with periodontitis. Prostaglandins are localized and potent mediators of numerous different physiologic processes and their production can be triggered by specific interleukin compounds. Tumor necrosis factor-alpha (TNF-α) is a cytokine produced by leukocytes during infection, and appears to have a significant role in bone resorption during osteomyelitis. TNF-α appears to regulate bone resorption in a different manner from arachidonic acid metabolites (such as prostaglandin E2 (PGE₂) and leukotrienes).

Likewise, oral care anti-inflammatory active ingredients can also play a role in reducing or scavenging reactive oxide species within the oral cavity. Reactive oxygen species (ROS) are also proinflammatory mediators that are typically highly reactive products produced during various biochemical processes, and include superoxide anions (O₂⁻), hydrogen peroxide (H₂O₂), and hydroxyl radicals (OH). The formation of ROS can occur as part of many cellular processes including mitochondrial respiration, immune cell responses, cell injury, heat, radiation of many origins, from metabolism of drugs and other chemicals. ROS are thought to be involved in almost all disease processes, as well as in the ageing process. Increased ROS formation under pathological conditions is believed to cause cellular damage through the action of these highly reactive molecules by crosslinking proteins, mutagenizing DNA, and peroxidizing lipids.

The suppression of one or more of the above described proinflammatory mediators prevents and/or treats tissue damage and/or tissue loss when the tissue is inflamed. Thus, preferred oral care active ingredients useful for oral compositions may serve as anti-inflammatory agents that suppress or reduce one or more mediators of inflammation. In certain embodiments, the contacting of the oral composition with the inflamed oral tissue further reduces formation of plaque and/or tartar on an oral surface. Thus, a method of treating inflammation in an oral tissue comprises contacting an oral composition having an active ingredient that comprises a tocopherol agent. In certain embodiments, the active ingredient further comprises a zinc citrate agent as described above. The tocopherol agent of the active ingredient may reduce and/ or suppress the production of one or more proinflammatory mediators to reduce inflammation in oral tissue.

As used herein, a "tocopherol agent" refers to any tocopherol compound deriving from a family of structurally similar 6-chromanol derivatives, enantiomers, racemates, or other analogues, including synthetic and naturally derived compounds. Such compounds include α-tocopherol ((+)-2,5,7,8-tetramethyl-2-(4,8,12-trimethyltridecyl)-6-chromanol), β-tocopherol ((+)-2,5,8-trimethyl-2-(4,8,12-trimethyltridecyl)-6-chromanol), γ-tocopherol ((+)-2,7,8-trimethyl-2-(4,8,12-trimethyltridecyl)-6-chromanol), δ-tocopherol ((+)-8-methyl-2-(4,8,12-trimethyltridecyl)-6-chromanol), α-tocotrienol (2,5,7,8-tetramethyl-2-(4,8,12-trimethyl-3,7,11-tridecatrienyl)-6-chromanol), β-tocotrienol (2,5,8-trimethyl-2-(4,8,12-trimethyl-3,7,11-tridecatrienyl)-6-chromanol), and tocol (2-methyl-2-(4,8,12-trimethyltridecyl)-6-chromanol). Generally, "Vitamin E" refers to biologically active compounds of the tocopherol family and can encompass a mixture of any two or more tocopherol and/or tocotrienol compounds listed above. Further, the tocopherol agent can comprise esterified and non-esterified forms of Vitamin E, for example, Vitamin E acetate ((+)-2,5,7,8-tetramethyl-2-(4,8,12-trimethyltridecyl)-6-chromanol-acetate) or d-Vitamin E acetate (2,5,7,8-tetramethyl-2-(4,8,12-trimethyltridecyl)-6-chromanol-acetate) both of which are derivatives of α-tocopheryl. While α-tocopherol is generally recognized as the most active form of Vitamin E, and is suitable for use in the present invention, in some embodiments, a mixture of different tocopherol family compounds is particularly efficacious.

In certain embodiments, an oral composition comprises a tocopherol agent that comprises at least two distinct compounds of the tocopherol family. Thus in certain embodiments, an active ingredient comprises at least two distinct tocopherol compounds chosen from tocol, α-tocopherol, β-tocopherol, γ-tocopherol, δ-tocopherol, α-tocotrienol, β-tocotrienol or derivatives or mixtures thereof. In other embodiments, the tocopherol agent comprises at least two compounds chosen from α-tocopherol, β-tocopherol, γ-tocopherol or δ-tocopherol. One such product is commercially available from Riken Vitamin Co., Ltd. (Tokyo, Japan) as a mixed tocopherol product that contains both synthetic and natural α, β, γ, and δ-tocopherol compounds. In other embodiments, the tocopherol agent comprises a mixture of α-tocopherol and γ-tocopherol. A mixture of tocopherol compounds is particularly efficacious as an anti-inflammatory agent. For example, a mixture of α and γ-tocopherol compounds has been found to be particularly efficacious as an oral care anti-inflammatory active ingredient, including high uptake into inflamed oral tissues, which has not previously been observed.

The tocopherol agent comprising the tocopherol compounds can be present in the oral compositions in an amount of about 0.001 % to about 5%, for example about 0.1% to about 2.5% or about 0.2% to about 1% by weight of the total composition. For example, in various embodiments where the tocopherol agent comprises mixtures of tocopherols, such as at least two distinct tocopherol compounds, e.g., α and γ-tocopherols, each respective tocopherol compound is present in an amount of about 0.1% to about 1%, about 0.2% to about 0.75%, or about 0.3% to about 0.6% by weight of the oral composition.

Additional optional oral care compounds that can be included as active ingredients in any of the oral compositions described above include, for example, additional antibacterial agents, whitening agents, additional anti-caries and tartar control agents, periodontal actives, abrasives, breath freshening agents, malodor control agents, tooth desensitizers, salivary stimulants, whitening agents and combinations thereof. Any given material may serve multiple purposes within two or more of such categories of actives. Exemplary actives among those useful herein are disclosed in U.S. Patent Nos. 4,894,220 to Nabi, et al., 5,288,480 to Gaffar, et al., U.S. Patent Publication No. 2003/0206874 to Doyle et al., as well as in U.S. Patent No. 6,290,933 to Durga et al., and U.S. Patent No. 6,685,921 to Lawlor.

Actives useful herein are optionally present in the oral compositions in safe and effective amounts. A "safe and effective" amount in the present context is an amount sufficient to provide a desired benefit, for example a therapeutic, prophylactic, nutritive or cosmetic effect, when the composition is used repeatedly as described herein, without undue side effects such as toxicity, irritation or allergic reaction, commensurate with a reasonable benefit/risk ratio. Such a safe and effective amount will usually, but not necessarily, fall within ranges approved by appropriate regulatory agencies. A safe and effective amount may depend on the particular benefit desired or condition being treated or sought to be prevented, the particular subject using, or being administered, the composition, the frequency and duration of use, *etc.* Actives are typically present in a total amount of about 0.01% to about 80%, for example about 0.05% to about 60%, about 0.1% to about 50%, or about 0.5% to about 40%, by weight of the composition.

Specifically, useful additional oral care compounds include, e.g., non-ionic antibacterial agents, including phenolic and bisphenolic compounds, such as, *e.g.,* halogenated diphenyl ethers, including triclosan (2,4,4'-trichloro-2'-hydroxy-diphenylether, triclocarban (3,4,4-trichlorocarbanilide), as well as 2-phenoxyethanol, benzoate esters, and carbanilides. Such additional antibacterial agents can be present in the oral care composition in an amount of about 0.01 to about 5% by weight of the oral composition. Useful anti-tartar agents in addition to the zinc ion sources and polyphosphates described above include tin ion sources, such as such as stannous fluoride, stannous chloride, and stannous pyrophosphate.

In some embodiments, the active ingredient may comprise a source of fluoride ions or fluorine-providing component, as anti-caries and/or anti-tartar agents, in amount sufficient to supply about 25 ppm to 5,000 ppm of fluoride ions. Fluoride ion sources comprise inorganic fluoride salts, such as soluble alkali metal salts; for example: sodium fluoride, potassium fluoride, sodium monofluorophosphate, sodium fluorosilicate, ammonium fluoro silicate, amine fluorides, including olaflur (N'-octadecyltrimethylenediamine-N,N,N'-tris(2-ethanol)-dihydrofluoride), as well as tin fluorides, such as stannous fluoride.

The oral composition may optionally comprise a nutrient such as a vitamin, mineral, anti-oxidant, and/or amino acid active compound. Useful nutrients include without limitation, vitamins including sources of vitamin C, including ascorbic acid; carotenoids, including retinol (vitamin A), retinal, retinoic acid, α-carotene, β-carotene, γ-carotene, δ-carotene, lutein, lycopene, lycophyll, lycoxanthin, rhodoxanthin, astaxanthin and cryptoxanthin; sources of B vitamins, including thiamine (vitamin B₁), riboflavin (vitamin B₂), nicotinamide and nicotinic acid (both referred to as niacin), pantothenic acid (vitamin B₅), pantothenol, pyridoxine (vitamin B₆), pyridoxal, pyridoxamine, folic acid, dihydrofolic acid, cyanocobalamin (vitamin B₁₂) and biotin; bioflavonoids, including rutin, hesperetin, hesperidin, eriodictyol, quercetin, quercetagetin and quercetagitrin; quinone-type enzyme cofactors, including ubiquinone (coenzyme Q₁₀), pyrroloquinoline quinone (PQQ), paraaminobenzoic acid; sources of α-lipoic acid; sources of vitamin D, including calciferol and cholecalciferol; salts, esters (including phosphate, acetate and long-chain, *e.g.,* linoleate and palmitate, esters), isomers, enantiomers, racemates and tautomers of the above. Nutritional supplements include amino acids (such as L-tryptophane, L-lysine, methionine, threonine, levocarnitine and L-carnitine), lipotropics (such as choline, inositol, betaine, and linoleic acid), fish oil (including components thereof such as omega-3 (N-3) polyunsaturated fatty acids, eicosapentaenoic acid and docosahexaenoic acid), and mixtures thereof.

The nutrient component can be a multivitamin complex comprising, in addition to a tocopherol agent, a plurality of vitamin/vitaminoids chosen from: (a) sources of vitamin C; (b) carotenoids; (c) sources of B vitamins; (d) bioflavonoids; (e) quinone-type enzyme cofactors; (f) sources of α-lipoic acid; or (g) sources of vitamin D.

In certain embodiments where the active ingredient comprises a tocopherol agent, the active ingredient may also comprise pantothenic acid/ pantothenol (vitamin B₅) or orally acceptable salts or esters thereof. The total concentration of sources of vitamin B₅ in an oral composition may be, in various embodiments, about 0.005% to about 1%, about 0.01% to about 0.5%, about 0.03% to about 0.1%, or about 0.05%.

Also described herein is an oral composition comprising a zinc salt, a linear polyphosphate salt having an average chain length of 3 or less, and at least one of a potassium salt or a vitamin. The vitamin may be any vitamin known in the art, including those enumerated in the present disclosure, and the potassium salt may be any potassium salt known in the art, including those enumerated in the present disclosure,. In certain embodiments, the linear polyphosphate salt is pyrophosphate, a tripolyphosphate or a tetrapolyphosphate.

The oral compositions may be provided in an orally acceptable carrier or vehicle. The carrier can be a liquid, semi-solid, or solid phase, in the form of a mouth rinse, dentifrice (including toothpastes, toothpowders, and prophylaxis pastes), confectionaries (including lozenges, and gum), medicament, film, or any other form known to one of skill in the art. Selection of specific carrier components is dependant on the desired product form.

In various embodiments, the oral composition has an orally acceptable vehicle that has a pH of about 6 to 10, or about 7 to 9. This particularly desirable in embodiments where the oral composition comprises components to reduce the astringency of the zinc citrate agent as described previously above, such as the polyphosphate compound. A lowering of the pH to less than about 6 can potentially result in precipitation of the zinc citrate, particularly when it is complexed with either the polyphosphate salt and/or the polycarboxylate polymer. Certain components serve to raise the pH of the oral composition. Such compounds include conventional buffers and salts, as well as chemicals such as the anionic linear polycarboxylates (described above) and polyacrylates such as those available from B.F. Goodrich of Cleveland, OH sold under the tradename CARBOPOL® have been observed to raise pH when present in oral compositions.

Conventional ingredients can be used to form the carriers listed above. The oral compositions may include other materials in addition to those components previously described, including for example, surface active agents, such as surfactants, emulsifiers, and foam modulators, viscosity modifiers and thickeners, humectants, diluents, additional pH modifying agents, emollients, moisturizers, mouth feel agents, sweetening agents, flavor agents, colorants, preservatives, solvents, such as water and combinations thereof. Any given material may serve multiple purposes within two or more of such categories of materials. Preferably, such carrier materials are selected for compatibility and stability with all of the constituents of the active ingredient.

Useful surface active agents are disclosed in the patent references referenced and discussed above, including in U.S. Patent No. 4,894,220. Surface active agents generally are an important aspect of the oral composition, as they can function as surfactants, emulsifiers foam modulators, and/or active ingredient dispersion agents. Thus, their selection for compatibility with the active ingredient constituents is important. For example, in embodiments where the oral composition has an active ingredient comprising a cationic antibacterial agent, it is preferred that the carrier comprises surfactants that are not strongly anionic, as such anionic compounds can bind to the cationic active ingredient potentially reducing its bioavailability.

Suitable surface active agents are those that are reasonably stable and foam throughout a wide pH range. These compounds are known in the art, and include non-soap anionic (*e.g.,* sodium lauryl sulfate (SLS), N-myristoyl, and N-palmitoyl sarcosine), nonionic (*e.g.,* Polysorbate 20 (polyoxyethylene 20 sorbitan monolaurate, TWEEN® 20) and Polysorbate 80 (polyoxyethylene 20 sorbitan monooleate, TWEEN® 80), Poloxamer 407, available under the trade name PLURONIC® F127 from BASF Corporation), cationic, zwitterionic (*e.g.,* cocoamidopropyl betaine and lauramido propyl betaine), and amphoteric organic synthetic detergents. In various embodiments, one or more surface active agents are present in the oral composition in the range of about 0.001% to about 5%, or about 0.5% to about 2.5%. In embodiments where the oral composition comprises an active ingredient comprising lipophilic active compound(s), the amount of surface active agent is increased to enable sufficient emulsification of the active ingredients within the carrier of the oral composition. The carrier is typically aqueous. For example, in embodiments where the tocopherol agent is present in an aqueous carrier, the amount of surface active agent (such as SLS) present may be present at about 1 to about 3% by weight of the oral composition, for example at about 1.5%.

In embodiments where the oral composition is in the form of a mouthrinse, an exemplary carrier is substantially liquid. The term "mouthrinse" includes mouthwashes, sprays and the like. In such a preparation the orally acceptable carrier typically has an aqueous phase comprising either water, or a water and alcohol mixture. Further, in various embodiments, the oral carrier typically contains a humectant, surfactant, and a pH buffering agent.

The oral composition may optionally comprise a flavoring agent. Exemplary flavoring substances are known to a skilled artisan, and may be present in certain embodiments at a concentration of about 0.05% by weight to about 5% by weight.

In embodiments where an oral composition is in the form of a confectionary, an exemplary carrier may be substantially solid or semi-solid. Confectionary carriers are known in the art. For a lozenge, the carrier typically comprises a lozenge base material (for example, comprising a non-cariogenic polyol and/or starch/sugar derivative), an emulsifier, a lubricant, a flavoring agent, a thickener, and optionally a coating material. Chewing gum carriers generally have a chewing gum base, one or more plasticizing agents, a sweetening agent, and a flavoring agent.

In embodiments where an oral composition is in the form of a film, an exemplary carrier is substantially solid or semi-solid. Generally, such film carriers comprise a water soluble or dispersible film forming agent, such as a hydrophilic polymer. Optionally, the film carrier may also comprise hydrophobic film forming polymers, either as a removable backing layer, or mixed with a hydrophilic film forming polymer. Film carriers optionally comprise plasticizers, surface active agents, fillers, bulking agents, and viscosity modifying agents.

In embodiments where an oral composition is in the form of a dentifrice, an exemplary carrier is substantially semi-solid or a solid. Dentifrices typically contain surface active agents, humectants, viscosity modifying agents and/or thickeners, abrasives, solvents, such as water, flavoring agents, and sweetening agents.

In embodiments where an oral composition is in the form of a medicament, such as a non-abrasive gel or ointment that can be applied to the gingival sulcus or margin and can be used in conjunction with wound dressings, gauze, films, and the like. Such gels may include both aqueous and non-aqueous gels. Aqueous gels generally comprise a polymer base, a thickener, a humectant, a flavoring agent, a sweetening agent, and a solvent, typically including water.

In various embodiments, the methods of the invention promote oral health in an oral cavity and treat plaque on an oral surface of a mammalian subject. In one embodiment, a method of providing one or more oral health benefits to an oral cavity of a mammalian subject entails preparing an oral composition as described herein, where an active ingredient comprises a zinc citrate agent and a second agent. The second agent is optionally potassium citrate or a tocopherol agent. The prepared oral composition is contacted with an oral surface within an oral cavity. The oral composition containing the active ingredient may provide multiple oral health benefits, such as anti-gingivitis, anti-periodontitis, anti-caries, anti-tartar, anti-inflammatory, analgesic, anti-aging, and breath freshening.

Thus, any of the various embodiments of the oral care composition described above are contacted with or applied regularly to an oral surface, preferably at least one time a day, more preferably on multiple days in a week, and most preferably on a long-term daily basis.

The oral composition of the present invention can be made by any of the methods known in the art for combining ingredients to make oral care compositions. Examples of methods that can be used are set forth in: U.S. Patent No. 6,403,059 to Martin et al., Clinical Pharmacology for Dental Professionals (Mosby-Year Book, Inc., 3rd ed. 1989); Mosby's Dental Hygiene: Concepts, Cases and Competencies, (Daniel Daniel, S., and Harfst, S., eds., Elsevier Science Health Science Div. 2002) and Ernest W. Flick, Cosmetic and Toiletry Formulations, 2nd ed.).

Dentifrices are typically prepared by adding various salts (including zinc and fluoride salts, when included in the composition), and sweeteners (*e.g.,* saccharin), and any water-soluble oral care active ingredient compounds to water, where it is mixed. Into another container, all humectants, gums, and polymers are added together. The water based mixture described above is added to the container with the humectants, gums, and polymers. The combined ingredients are optionally heated to a temperature of greater than about 40°C, for example from about 60°C to about 70°C, to disperse the gums and polymers. The heated mixture is then cooled to less than approximately 38°C (about 100°F). The mixture is then combined with abrasives, where it is mixed at high speed under a vacuum for about 15 to about 20 minutes. Any lipophilic active ingredients are admixed into flavor oil (and/or alcohol). This mixture is admixed to the water based mixture above, where it is mixed under high speed and vacuum until sufficiently dispersed. The surfactant(s) are added and the mixture is again mixed to disperse.

In certain embodiments, a method of making an oral composition comprises adding an additional active compound as part of the active ingredient to the one or more carrier ingredients prior to admixing. In other embodiments, such additional oral care active ingredients are added with lipophilic ingredients to the homogenous mixture. Whether additional oral care actives are added to the one or more carrier ingredients prior to admixing them to form a homogenous mixture, or added to the mixture with the lipophilic components after admixing, is dependent upon the nature of the additional active ingredient (for example, whether it can withstand heating to greater than or equal to about 40 °C and whether it is hydrophobic, hydrophilic, anionic, cationic, or non-ionic). One of skill in the art can readily determine the appropriate point in the method of making the oral composition to add the active ingredients, based upon these considerations. For example, in certain embodiments, where the oral care active ingredient comprises a source of soluble zinc ions and fluoride ions, the soluble zinc salts and/ or fluoride salts can be added to the one or more carrier ingredients prior to the admixing because they are substantially soluble in water.

The present invention is further illustrated through the following nonlimiting example(s).

### Example 1

Dentifrice compositions of which dentifrices 1 and 2 are not in accordance with the present invention, and dentifrice 3 is a composition in accordance with the present invention) are made by combining the following ingredients:

| INGREDIENTS | DENTIFRICE 1 | DENTIFRICE 2 | DENTIFRICE 3 |
|---|---|---|---|
| Sorbitol (70% aqueous solution) | 10-20 | 55-65 | 15-30 |
| Synthetic Precipitated | 15-23 | 24-30 | --- |
| Hydrated Silica | --- | --- | 10-18 |
| Polyethylene Glycol 600 | 1-5 | 1-5 | 1-5 |
| Glycerin | 10-15 | --- | 8-12 |
| GANTREZ® | 1-2 | --- | 1-2 |
| Sodium Lauryl Sulfate | 1-2 | 1-2 | 1-2 |
| Tetrasodium Pyrophosphate | 2-3 | 0.1-2 | 2-3 |
| Xanthan Gum | --- | --- | 0.01-1 |
| Sodium Sulfate | 0.1-2 | --- | 0.01-1 |
| Sodium Hydroxide | 0.1-2 | --- | --- |
| Sodium CMC | 0.5-3 | 0.5-3 | 0.1-3 |
| Potassium Hydroxide | | | 0.1-2 |
| Flavor | 0.1-3 | 0.1-3 | 0.5-3 |
| Color | 0.01-2 | 0.01-2 | 0.0001-0.01 |
| Titanium Dioxide | 0.5-3 | --- | --- |
| Carrageenan | --- | 0.1-1 | --- |
| Sodium Saccharin | 0.01-1 | 0.01-1 | 0.01-1 |
| Sodium Fluoride | --- | 0.01-1 | --- |
| Sodium Monofluorophosphate | 0.5-2 | --- | 0.5-2 |
| Vitamin E | 0.1-1 | 0.1-2 | --- |
| Mixed Tocopherols | --- | 0.1-2 | --- |
| Zinc Citrate | 1-5 | --- | 1-5 |
| Potassium Citrate | --- | --- | 3-8 |
| Panthenol (Vitamin B₅) | 0.01-1 | --- | --- |
| Water | Q.S. | Q.S. | Q.S. |

Dentifrice 1 corresponds to an oral composition comprising active ingredients including a zinc citrate agent, an α-tocopherol agent (Vitamin E), a panthenol (Vitamin B₅) agent, and a fluoride providing source (sodium monofluorophosphate (MFP)), that provides an anti-plaque, anti-tartar, anti-caries, and anti-inflammatory oral composition. Dentifrice 2 comprises an active ingredient comprising at least two tocopherol compounds, namely an α-tocopherol agent (Vitamin E) and a Mixed Tocopherol agent comprising α, β, δ, γ-tocopherol compounds, as well as a fluoride providing source (sodium fluoride). Dentifrice 2 provides anti-inflammatory and anti-caries benefits, *inter alia.* Dentifrice 3 is an oral composition comprising active ingredients including a zinc citrate agent, a potassium citrate agent, and a fluoride source (sodium MFP), which provides a reduction in dentinal sensitivity, an anti-plaque, anti-tartar and anti-caries benefit.

## Claims

1. An antiplaque and desensitizing oral composition comprising:
an active ingredient comprising a zinc citrate agent and a potassium citrate agent; and
a copolymer of maleic anhydride and vinyl methyl ether, and a polyphosphate.

2. The composition according to claim 1, wherein the zinc citrate agent comprises a compound having a general nominal formula of Zn₃(C₆H₅O₇)₂.

3. The composition according to claim 1, wherein the potassium citrate agent comprises a compound having a general nominal formula of K₃C₆H₅O₇.

4. The composition according to claim 1, wherein the active ingredient further comprises a compound chosen from potassium tartrate, potassium chloride, potassium sulphate, potassium nitrate, sodium nitrate, sodium citrate or mixtures thereof.

5. The composition according to claim 1, wherein a ratio of the zinc citrate agent to the potassium citrate agent is about 0.5:1 to about 1:0.5.

6. The composition according to claim 1, wherein the zinc citrate agent is present in an amount of about 0.001% to about 5% by weight of the oral composition and the potassium citrate agent is present in an amount of about 0.001% to about 10% by weight of the oral composition.

7. The composition according to claim 1, wherein the oral composition further comprises a non-ionic halogenated diphenyl ether.

8. The composition according to claim 1, wherein the oral composition is in a form of a mouth rinse, a dentifrice, a confectionary, a medicament, or a film.

9. An oral composition according to any one of claims 1 to 8 for use in a method of treating an oral surface having dental sensitivity, the method comprising contacting the oral surface with the oral composition.

10. The oral composition according to claim 9, wherein the oral composition further comprises a tocopherol agent.

11. The oral composition according to claim 9, wherein the oral composition has a pH of about 6 to about 10.

## Patentansprüche

1. Antiplaque- und Desensibilisierungs-Oralzusammensetzung umfassend:
einen aktiven Bestandteil umfassend ein Zinkzitratmittel und ein Kaliumzitratmittel, und
ein Copolymer aus Maleinsäureanhydrid und Vinylmethylether, und ein Polyphosphat.

2. Zusammensetzung nach Anspruch 1, worin das Zinkzitratmittel eine Verbindung mit der allgemeinen Nominalformel Zn₃(C₆H₅O₇)₂.

3. Zusammensetzung nach Anspruch 1, worin das Kaliumzitratmittel einer Verbindung mit der allgemeinen Nominalformel K₃C₆H₅O₇ umfasst.

4. Zusammensetzung nach Anspruch 1, worin der aktive Bestandteil weiterhin eine Verbindung umfasst, ausgewählt aus Kaliumtartrat, Kaliumchlorid, Kaliumsulfat, Kaliumnitrat, Natriumnitrat, Natriumzitrat oder Mischungen davon.

5. Zusammensetzung nach Anspruch 1, worin das Verhältnis des Zinkzitratmittels zum Kaliumzitratmittel etwa 0,5:1 bis etwa 1:0,5 ist.

6. Zusammensetzung nach Anspruch 1, worin das Zinkzitratmittel in einer Menge von etwa 0,001 Gew.-% bis etwa 5 Gew.-% der Oralzusammensetzung vorliegt und das Kaliumzitratmittel in einer Menge von etwa 0,001 Gew.-% bis etwa 10 Gew.-% der Oralzusammensetzung vorliegt.

7. Zusammensetzung nach Anspruch 1, worin die Oralzusammensetzung weiterhin einen nicht-ionischen halogenierten Diphenylether umfasst.

8. Zusammensetzung nach Anspruch 1, worin die Oralzusammensetzung in Form einer Mundspülung, eines Zahnreinigungsmittels, eine Konfekts, eines Medikaments, oder eines Films ist.

9. Oralzusammensetzung gemäß irgendeinem der Ansprüche 1 bis 8 zur Verwendung in einem Verfahren zur Behandlung einer oralen Oberfläche mit dentaler Empfindlichkeit, wobei das Verfahren das in In-Kontakt-Bringen der oralen Oberfläche mit der Oralzusammensetzung umfasst.

10. Oralzusammensetzung nach Anspruch 9, worin die Oralzusammensetzung weiterhin ein Tocopherol-Mittel umfasst.

11. Oralzusammensetzung nach Anspruch 9, worin die Oralzusammensetzung einen pH-Wert von etwa 6 bis etwa 10 hat.

## Revendications

1. Composition orale anti-plaque et désensibilisante comprenant :
un principe actif comprenant un agent citrate de zinc et un agent citrate de potassium ; et
un copolymère d'anhydride maléique et d'éther de vinyle et de méthyle, et un polyphosphate.

2. Composition selon la revendication 1, dans laquelle l'agent citrate de zinc comprend un composé ayant une formule générale nominale de Zn₃(C₆H₅O₇)₂.

3. Composition selon la revendication 1, dans laquelle l'agent citrate de potassium comprend un composé ayant une formule générale nominale de K₃C₆H₅O₇.

4. Composition selon la revendication 1, dans laquelle le principe actif comprend en outre un composé choisi parmi le tartrate de potassium, le chlorure de potassium, le sulfate de potassium, le nitrate de potassium, le nitrate de sodium, le citrate de sodium ou les mélanges de ceux-ci.

5. Composition selon la revendication 1, dans laquelle un rapport de l'agent citrate de zinc à l'agent citrate de potassium est d'environ 0,5:1 à environ 1:0,5.

6. Composition selon la revendication 1, dans laquelle l'agent citrate de zinc est présent en une quantité d'environ 0,001 % à environ 5 % en poids de la composition orale et l'agent de citrate de potassium est présent en une quantité d'environ 0,001 % à environ 10 % en poids de la composition orale.

7. Composition selon la revendication 1, dans laquelle la composition orale comprend en outre un éther de diphényle halogéné non ionique.

8. Composition selon la revendication 1, dans laquelle la composition orale se présente sous la forme d'un rince-bouche, d'un dentifrice, d'une confiserie, d'un médicament ou d'un film.

9. Composition orale selon l'une quelconque des revendications 1 à 8 pour une utilisation dans une méthode de traitement d'une surface buccale présentant une sensibilité dentaire, la méthode comprenant la mise en contact de la surface buccale avec la composition orale.

10. Composition orale selon la revendication 9, dans laquelle la composition orale comprend en outre un agent tocophérol.

11. Composition orale selon la revendication 9, dans laquelle la composition orale a un pH d'environ 6 à environ 10.
